Europäisches Patentamt

European Patent Office

Office européen des brevets

(11) Publication number: **0 245 098 B1**

# EUROPEAN PATENT SPECIFICATION

(45) Date of publication of patent specification: **01.12.93**   (51) Int. Cl.5: **A61B 5/103**, G06F 15/42

(21) Application number: **87304069.5**

(22) Date of filing: **07.05.87**

(54) **Method and apparatus for judging deformation of vertebral body.**

(30) Priority: **07.05.86 JP 103198/86**
     **31.07.86 JP 178882/86**

(43) Date of publication of application:
     **11.11.87 Bulletin 87/46**

(45) Publication of the grant of the patent:
     **01.12.93 Bulletin 93/48**

(84) Designated Contracting States:
     **CH DE FR GB IT LI**

(56) References cited:
     **EP-A- 0 069 229**

     **MEDICAL & BIOLOGICAL ENGINEERING &
     COMPUTING, vol. 20, no. 6, November 1982,
     pages 715-726, Stevenage, Herts, GB; J.
     KORESKA et al.: "Portable desktop
     computer-aided digitiser system for the ana-
     lysis of spinal deformities"**

(73) Proprietor: **TEIJIN LIMITED**
     **11 Minami Honmachi 1-chome**
     **Higashi-ku**
     **Osaka-shi Osaka-fu(JP)**

(72) Inventor: **Yamashita, Gentaro**
     **4-10-7, Shibazaki-cho**
     **Tachikawa-shi Tokyo(JP)**
     Inventor: **Uotani, Yasuhiro**
     **2-92-1, Shinmei-cho**
     **Koshigaya-shi Saitama(JP)**
     Inventor: **Aoki, Choju**
     **1-6-5, Nagasawa**
     **Tama-ku**
     **Kawasaki-shi Kanagawa(JP)**

(74) Representative: **Arthur, Bryan Edward et al**
     **4 Dyers Buildings**
     **Holborn**
     **London, EC1N 2JT (GB)**

**Description**

The present invention relates to a method and apparatus for judging deformation of a vertebral body. More particularly, the present invention relates to a method and apparatus for judging deformation of a vertebral body which is one of the bones forming the spinal column. A judgment of the presence of vertebral body deformation accompanied by osteoporosis, as well as a classification of the deformation, is very important for grasping the progress of osteoporosis and for confirmation of the effect of therapy.

In the prior art, the presence of deformation of a vertebral body which is one of the bones forming the spinal column has been judged by a physician by visual observation of the profile X-ray images of thoracic vertebrae or lumbar vertebrae. However, compared with a fracture of the so called long bones such as the femur, tibia, radius, ulna, etc., a judgment of vertebral deformation is difficult since this is usually a wedge-shaped deformation, pressure fracture, depressed fracture, etc., and any judgment inevitably involves individual differences. Also, even if an attempt is made to monitor changes over a period of time, for confirmation of the effect of therapy, because the deformation cannot be quantified, progress of the deformation, if any, can not be easily determined.

EP-A 0069229 discloses a method for reducing the effect of patient movement in X-ray diagnosis, in which different images are derived from a video signal containing a sequence of images of a patient under examination, first and subsequent images contained in the signal being stored and used to form a secondary mask which is then subtracted from images contained in the video signal. A system for the analysis of spinal deformities is known from Medical & Biomedical Engineering & Computing 1982, 20, 715-726 in which a deformity in the curvature of the spine as a whole is measured by the digitization of X-ray data.

Also, although a method has been reported in which the ratio (a/d) of the central length (a) to the front brim length (d) of the third lumbar vertebrae is determined as the index (Lumbar Spine Score) for the degree of bone atrophy, or a change in the longitudinal and lateral bone beams of the third lumbar vertebrae is observed as the index for the degree of bone atrophy (severity classified by Jikei University), or a judgment of the fracture of a vertebral body is made by measurement of the central length (a), front brim length (d) and rear brim length (c) of a vertebral body [The New England Journal of Medicine, vol. 306, 446 (February 25, 1982)], a method for classifying the type of deformation of a vertebral body has not heretofore been known.

Accordingly, the objects of the present invention are to eliminate the above-mentioned disadvantages of the prior art and to provide a method for judging deformation of a vertebral body in a subject, characterised in that the method comprises the steps of:

(i) measuring a central length (a), a rear brim length (c) and a front brim length (d) from a plurality of profile X-ray images obtained from vertebral bodies having substantially no deformation at a portion corresponding to a portion of said vertebral body to be judged, storing the measurements in a memory, and determining values of c/d, a/c and a/d, the average values $\bar{c}$ and $\bar{d}$ of the rear brim lengths (c) and front brim lengths (d), respectively, and the standard deviations $\sigma_c$ and $\sigma_d$ of the values c and d, respectively:

(ii) measuring a, c, and d from a plurality of the profile X-ray images obtained from vertebral bodies having at least one deformation, storing the measurements in said memory, and determining values of c/d, a/c, a/d, and $\bar{c}$, $\bar{d}$, $\sigma_c$, and $\sigma_d$;

(iii) preparing a judgement standard from the data obtained in steps (i) and (ii) for determining the nature of the deformity according to a classification consisting of "no deformation" vertebrae, "wedge-shaped" vertebrae, "inverse wedge-shaped" vertebrae, "fish" vertebrae, and "flat" vertebrae;

(iv) measuring a, c, and d from a profile X-ray image of said vertebral body to be judged, storing the measurements in said memory, and determining c/d, a/c, and a/d; and

(v) classifying deformation of the vertebral body to be judged from c/d in the case of "wedge-shaped" and "inverse wedge-shaped" vertebrae, from $\bar{c}$, $\bar{d}$, $\sigma_c$, $\sigma_d$, and c/d in the case of "flat" vertebrae, and from $\bar{c}$, $\bar{d}$, $\sigma_c$, $\sigma_d$, a/c, a/d, and c/d in the case of the "no deformation" and "fish" vertebrae, in order to judge deformation and to monitor the effect of therapy on said vertebral body to be judged.

In accordance with the present invention, there is further provided apparatus for judging deformation of a vertebral body according to the method of claim 1, the apparatus being defined in claim 7.

The present invention will be better understood from the description set forth below with reference to the accompanying drawings, in which:

Figure 1 shows a profile X-ray image of a vertebral body;

Figure 2 shows the relationships of the respective vertebral body deformations and front brim length (d), rear brim length (c), etc., according to the judgment method of the present invention;

Figure 3 shows an example of vertebral body deformation;

Figure 4 shows the front brim length, central length, rear brim length, and vertebral body width in a profile X-ray image of a vertebral body;

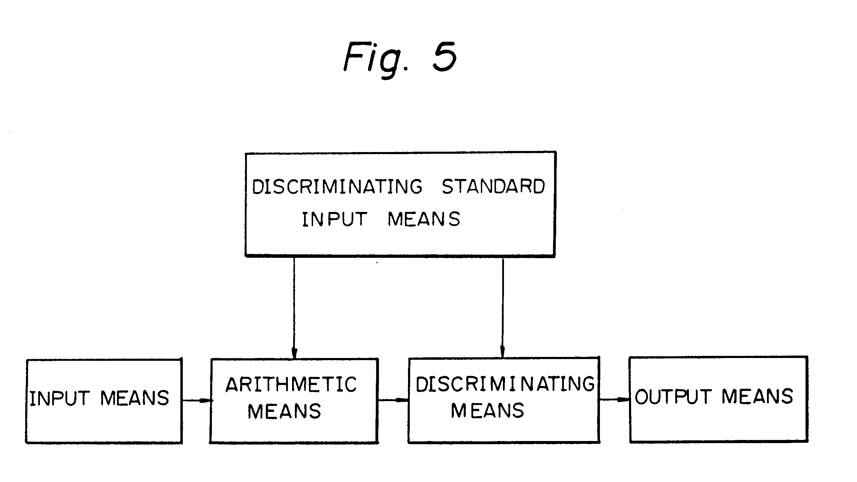

Figure 5 is a block diagram illustrating the basic structure of apparatus for judging deformation of a vertebral body by using an X-ray image of the vertebral body;

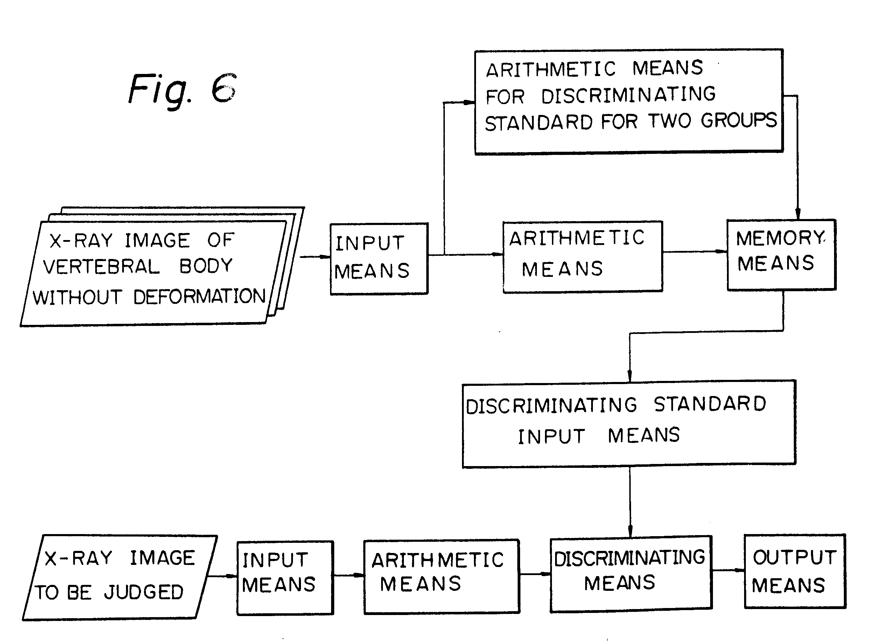

Figure 6 is a block diagram illustrating apparatus for judging deformation of a vertebral body in the X-ray image of the vertebral body to be judged by using a discriminating standard obtained mainly from X-ray images of vertebral bodies having no substantial deformation, in which a means for inputting information relating to X-ray images of vertebral bodies having deformation caused therein is further required;

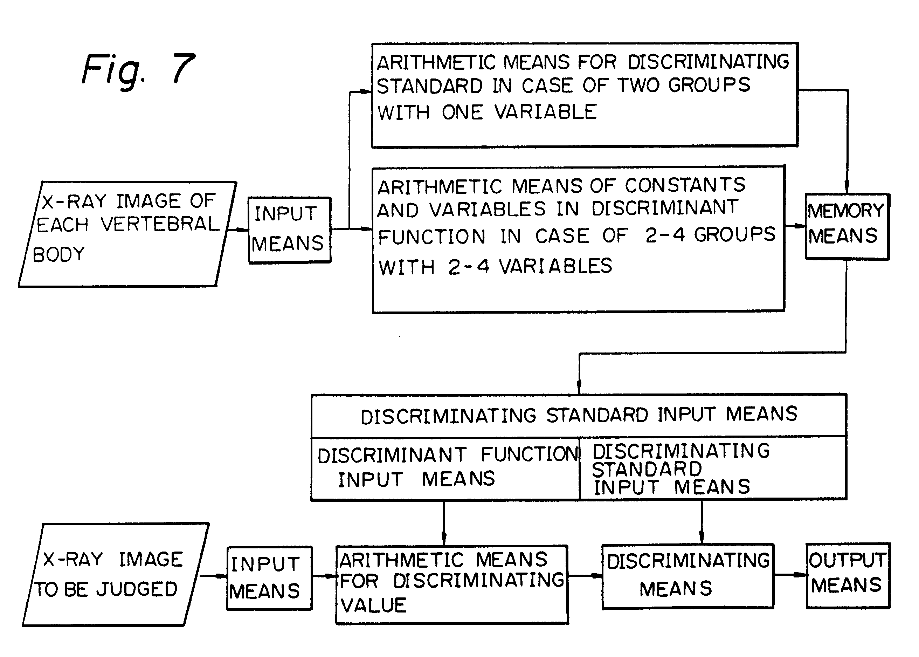

Figure 7 is a block diagram illustrating apparatus for judging deformation of a vertebral body by using, as a criteria, a discriminating standard obtained mainly by using a discriminating function;

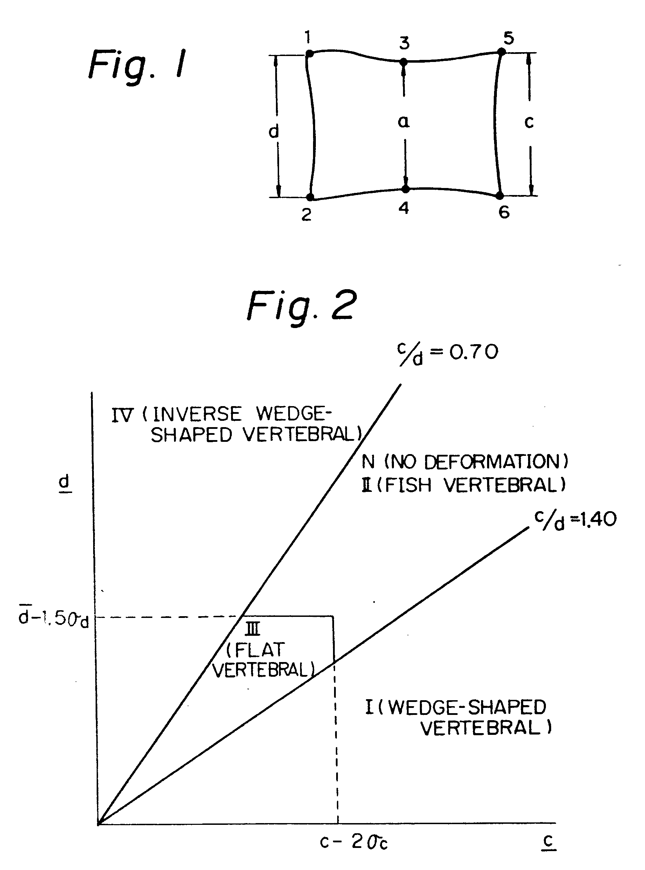

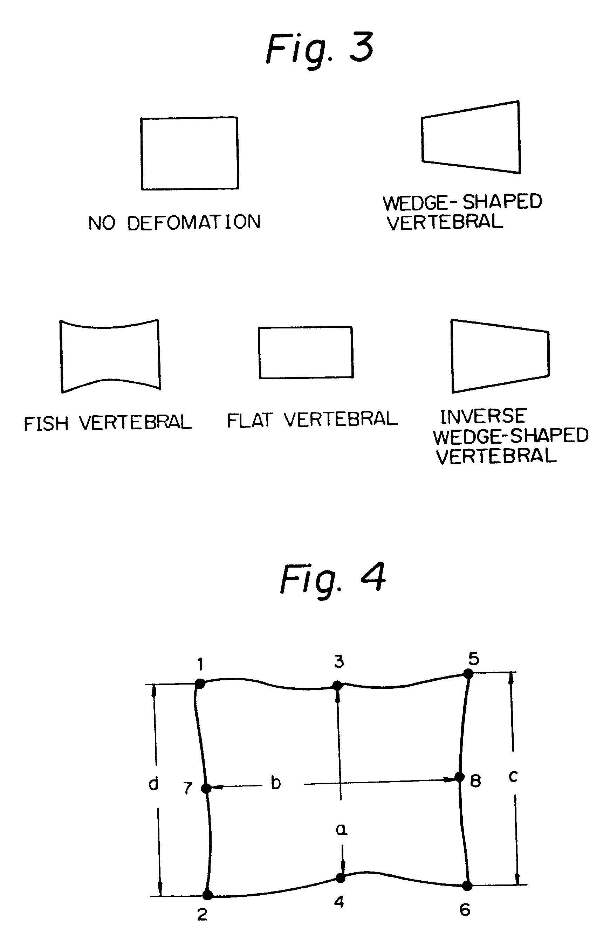

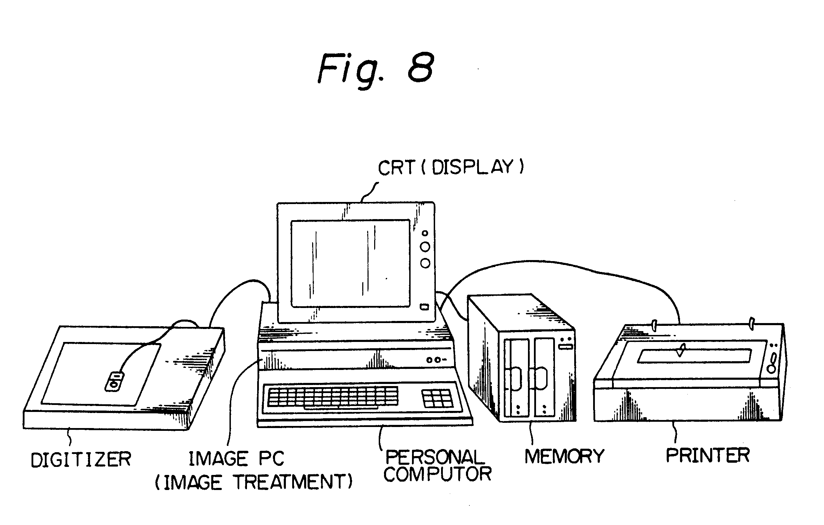

Figure 8 shows apparatus for judging deformation of a vertebral body comprising a digitizer for inputting the points 1 to 6 in Figure 1 or the points 1 to 8 in Figure 4 from the X-ray image to a computer; a personal computer for classifying the presence or absence of the deformation and types of the deformation by measuring the lengths $a$, $c$, and $d$ in Figure 1 or $a$, $b$, $c$ and $d$ in Figure 4, followed by the image treatment; a memory including software for effecting the abovementioned treatment; and a printer for printing out, for example, the measured values or the types of deformation; and

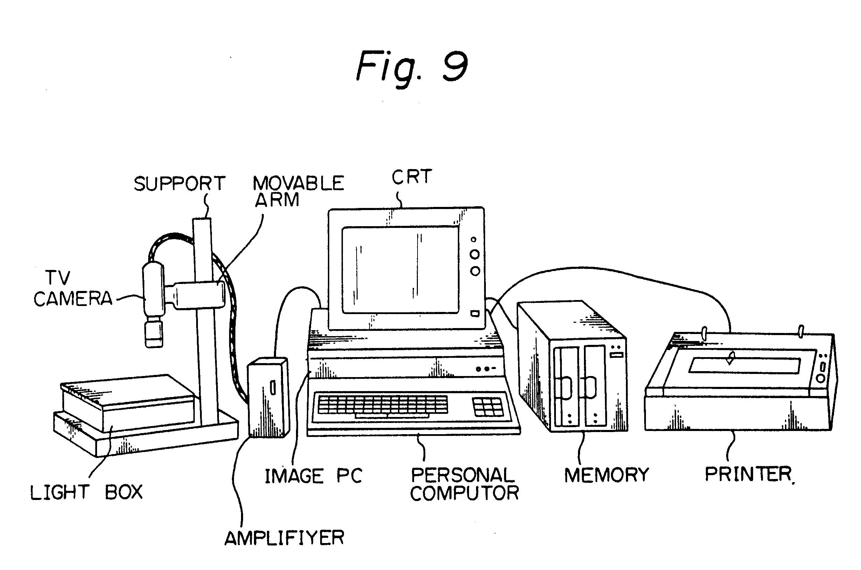

Figure 9 shows apparatus for judging deformation of a vertebral body comprising a TV camera for reading the image of each vertebral body from the X-ray image; a personal computer for classifying the presence or absence of the deformation and the types of the deformation by measuring the length $a$, $c$, and $d$ in Figure 1 or the lengths $a$, $b$, $c$, and $d$ in Figure 4 from the read X-ray image; a memory including software for registering the above images and effecting the abovementioned treatment.

The present inventors have made an intensive study of a method of objective evaluation of vertebral body deformation, and found that the presence of vertebral body deformation can be objectively judged according to the judgment standard in which lengths of the vertebral body and their ratios are combined by accurately measuring the lengths of a front brim, rear brim, central portion from a profile X-ray image (i.e., an image on a film taken from the side by X-ray radiation) of a vertebral body and determining the ratios of the respective portions of the vertebral body, and further found that the type of vertebral body deformation can be judged according to the judgment standard in which lengths of the vertebral body and their ratios are combined, and the progress over a period of time of the vertebral body deformation can be judged from the change in the deformation type as well as a change in the lengths of the vertebral body, and thus accomplished the present invention.

In the prior art, the profile X-ray image of vertebral body was visually observed by a physician when judging the vertebral body deformation, but according to the present invention, the lengths of the respective portions of the vertebral body are measured, judgment standards are prepared with reference to a judgment by a physician, and the presence of vertebral body deformation as well as a classification of the deformation type are objectively performed following the judgment standards.

The judgment method of the present invention is now described in more detail.

(i) First, from a profile X-ray of a vertebral body without deformation, the central length (a), rear brim length (c), and front brim length (d) of each vertebral body are measured to determine an average value $(\bar{c})$ of c, an average value $(\bar{d})$ of d, and a standard deviation $(\sigma_c , \sigma_d)$.

The above measurement is used to obtain standard values which become the judgment standard for the central length (a), rear brim length (c), and front brim length (d), etc., determined from a patient whose vertebral body deformation is to be judged.

The vertebral body without deformation to be measured is preferably a vertebral body of a person of from 50 to 75 years old, in view of the age of the patient whose vertebral body deformation is to be judged. Also, it is preferable to perform measurements separately for men and women, and it is desirable to perform measurements of about 50 vertebral bodies, or more, for both men and women.

When making a profile X-ray image of a vertebral body, the thoracic vertebrae from the first thoracic vertebrae to the twelfth thoracic vertebrae, lumbar vertebrae from the first lumbar vertebrae to the fifth lumbar vertebrae should be included, giving a total of 17 vertebral bodies, and therefore, for example, it is advantageous to take the photograph of the profile X-ray image with the eighth thoracic vertebrae as the center separately from the profile X-ray image with the third lumbar vertebrae as the center. In the profile X-ray image with the eighth thoracic vertebrae as the center, the first thoracic vertebrae cannot be accurately measured in most cases, and further, the second and the third thoracic vertebrae are unclear in many cases. However, since the frequency of vertebral body deformation is not great in these vertebral bodies, it is sufficient if measurement can be made from the fourth thoracic vertebrae or the fifth thoracic vertebrae.

3

The center length (a), rear brim length (c), and front brim length (d) of a vertebral body are specifically as shown in Fig. 1. For determination of these values, for the profile X-ray image of a vertebral body, the central length (a), rear brim length (c), and front brim length (d) may be measured for each vertebral body with a scale or slide calipers, but the six points of 1 to 6 shown in Fig. 1 can be input through a digitizer into a computer to measure the central length (a) (3-4), rear brim length (c) (5-6), and front brim length (d) (1-2), and to calculate the ratios of c/d, a/c, a/d. Also, for example, the degree of blackening may be recorded with a TV camera, etc., and the lengths of the respective portions of the vertebral body may be automatically measured by image processing.

(ii) Subsequently, from the profile X-ray image of the vertebral body of the patient whose vertebral body deformation is to be judged, as in (i), the center length (a), rear brim length (c), and front brim length (d) of each vertebral body are measured, and further, the ratios of c/d, a/c and a/d are determined by calculation.

These measurements can be conducted exactly as in the case of the above (i).

(iii) Next, a judgment of vertebral body deformation can be made from $\underline{a}$, $\underline{c}$, $\underline{d}$, c/d, a/c and a/d, for example, as follows.

That is, (A) when c/d, $\underline{c}$, $\underline{d}$, a/c, a/d satisfy the following conditions, the judgment can be "no deformation" (type N):

(a) 0.7 < c/d < 1.4;

(b) at least one of $c \geq \bar{c} - 2\sigma$ and $d \geq \bar{d} - 1.5\sigma$ is satisfied;

(c) at least one of $\overline{a/c} > 0.8$ and $\overline{a/d} > 0.8$ is satisfied.

As the judgment standard for such "no deformation", the ratio c/d of the rear brim length to the front brim length must be between 0.7 and 1.4 as the first condition (a). For, as described below, if c/d becomes 1.4 or more, the front brim portion becomes a deformed wedge-shaped vertebrae, and if c/d becomes 0.7 or less, the rear brim portion becomes a deformed inverse wedge-shaped vertebrae, which in practice is very rare.

Next, as the second condition (b), at least one of rear brim length c and front brim length d must be greater than $\bar{c} - 2\sigma$ and $\bar{d} - 1.5\sigma$ when the average values of the rear brim length c and front brim length d of vertebral body without deformation determined in (i) are made $\bar{c}$ and $\bar{d}$, respectively. In a typical healthy vertebral body, a, c, d all have a high value, namely $a \geq \bar{a} - 2\sigma_a$, $c \geq \bar{c} - 2\sigma_c$, $d \geq \bar{d} - 1.5\sigma_d$, but even when the vertebral body is slightly compressed to become $\bar{a} \geq \bar{a} - 2\sigma_a$, provided that at least one of the conditions of $c \geq \bar{c} - 2\sigma_c$ or $d \geq \bar{d} - 1.5\sigma_d$, can be satisfied, no clear vertebral body deformation is recognized and judgment of "no deformation" may be made.

As the third condition, at least one of a/c and a/d must be more than 0.8. For, if both of a/c and a/d are 0.8 or less, namely the center length a is much smaller, than the rear brim length $\underline{c}$ and front brim length $\underline{d}$, a fish vertebrae condition as described below is determined.

Thus, for the above reasons, when the above conditions of (a), (b) and (c) are satisfied, a judgment of "no deformation" can be made.

The relationship of the conditions of the above (a), (b), and (c), or the respective conditions as described below with the respective types of vertebral body deformation, inclusive of "no deformation", are shown in Fig. 2.

(B) When $c/d \geq 1.4$, the judgment can be a "wedge-shaped vertebrae" (type I).

In the wedge-shaped vertebrae, deformation has occurred at the front brim portion as shown in Fig. 3, and the front brim length (d) has become much smaller than the rear brim length (c). For example, when the front brim length is 3/4 of the rear brim length (c/d = 1.33), a wedge-shaped vertebral body deformation can be easily recognized, but in some cases, a clear wedge-shaped vertebrae cannot be easily recognized. On the other hand, when the front brim length is 2/3 of the rear brim length (c/d = 1.5), it can be clearly recognized as a wedge-shaped vertebrae, and therefore, $c/d \geq 1.4$ is made the judgment standard for wedge-shaped vertebrae.

In addition to the so called wedge-shaped vertebrae, the wedge-shaped vertebrae (type I) is inclusive of deformations with shortened front brim length, like a wedge-shaped vertebrae, as a result of, for example, an upper brim pressure fracture, upper brim depressed fracture, lower brim pressure fracture, lower brim depressed fracture, and as described below, even in the case of a vertebral body which is judged as a flat vertebrae because $a < \bar{a} - 2\sigma_a$, $c < \bar{c} - 2\sigma_c$, $d < \bar{d} - 1.5\sigma_d$, if there is a remarkable deformation at the front brim portion satisfying $c/d \geq 1.4$, it can be judged to be a wedge-shaped vertebrae.

(C) When $c/d \leq 0.7$, the judgment can be an "inverse wedge-shaped vertebrae" (type IV).

The inverse wedge-shaped vertebrae may be defined as a vertebral body wherein deformation has occurred at the rear brim portion, and thus the rear brim length (c) has become smaller than the front brim length (d), contrary to the wedge-shaped vertebrae wherein deformation has occurred at the front brim portion, and the front length (d) has become smaller than the rear brim length (c) (see Fig. 3), but in practice, such a vertebral body does not substantially exist. In the fifth lumbar vertebrae, as shown in Example 1, the rear brim length (c) is smaller than the front brim length (d), with the average value of c/d being lower than 1, e.g., 0.95, but in some cases, an inverse wedge-shaped vertebrae cannot be clearly recognized even when the rear brim length (c) is 3/4 of the front brim length (d) (c/d = 0.75), and therefore, a judgment of an inverse wedge-shaped vertebrae (type IV) can be made when $c/d \leq 0.7$.

(D) When c, d, c/d satisfy the conditions shown below, the judgment can be a "flat vertebrae" (type III):

    (a) $c < \bar{c} - 2\sigma_c$;

    (b) $\bar{d} < \bar{d} - 1.5\sigma_d$;

    (c) $0.7 < c/d < 1.4$.

In the first condition (a) and the second condition (b), the rear brim length (c) and the front brim length (d) are both smaller than $\bar{c} - 2\sigma_c$ and $\bar{d} - 1.5\sigma_d$ when the average values of the vertebral body without deformation are made $\bar{c}$ and $\bar{d}$; namely, $c < \bar{c} - 2\sigma_c$ and $d < \bar{d} - 1.5\sigma_d$.

The flat vertebrae is a vertebral body having a relatively uniform deformation at the front brim portion, the central portion, and the rear brim portion under pressure (see Fig. 3); namely, all of the front brim length (d), the central length (a) and the rear brim length (c) are smaller. As shown by the average values of the lengths of the respective portions of the vertebral body without deformation of Example 1, since the central length (a) is smaller than the front brim length (d) or the rear brim length (c) even in the vertebral body without deformation, the specific feature of the flat vertebrae resides in a particularly small front brim length (d) and rear brim length (c), and the vertebral body wherein $c < \bar{c} - 2\sigma_c$ and $d < \bar{d} - 1.5\sigma_d$ is judged to be a flat vertebrae (type III). The conditions of $-2\sigma_c$ for c and $1.5\sigma_d$ for d are set because $\bar{c}$ is greater than $\bar{d}$ ($\bar{c} > \bar{d}$) as shown in Example 1, and thus c and d become approximately equal values by setting such conditions, to satisfy the conditions for a flat vertebrae. The second condition (c) is $0.7 < c/d < 1.4$. For, even where $a < \bar{a} - 2\sigma_a$, $c < \bar{c} - 2\sigma_c$, $d < \bar{d} - 1.5\sigma_d$, deformation at the front brim portion is particularly marked, and where $c/d \geq 1.4$, it can be judged to be a wedge-shaped vertebrae, while in the case of a marked deformation at the rear brim portion when $c/d \leq 0.7$, it is judged to be an inverse wedge-shaped vertebrae.

(E) When c/d, c, d, a/c, a/d satisfy the following conditions, the judgment can be a "fish vertebrae" (type II):

    (a) $0.7 < c/d < 1.4$;

    (b) at least one of $c \geq \bar{c} - 2\sigma_c$ and $d \geq \bar{d} - 1.5\sigma_d$ is satisfied;

    (c) $a/c \leq 0.8$ and $a/d \leq 0.8$.

"Fish vertebrae" is a vertebrae where a depressed fracture or a pressure fracture has occurred at the central portion, whereby the central length (a) is particularly smaller than the front brim length (d) and rear brim length (c) (see Fig. 3). Accordingly, among the so called "no deformation" classifications, except for wedge-shaped vertebrae, flat vertebrae, and inverse wedge-shaped vertebrae, the central length (a) is particularly smaller. When the central length (a) is smaller than the front brim length (d) and the rear brim length (c), thus satisfying $a/c < 0.9$ and $a/d < 0.9$, deformation such as fish vertebrae can be clearly recognized, but in some cases a clear fish vertebrae cannot be recognized, and therefore, $a/c < 0.8$ and $a/c < 0.8$ can be made the judgment standards for fish vertebrae.

In addition to the so called fish vertebrae, the fish vertebrae (type II) is also inclusive of deformations with a shortened central length like fish vertebrae, as a result of an upper brim pressure fracture, upper brim depressed fracture, lower brim pressure fracture, lower brim depressed fracture, etc.

The relationships between these judgment standards for "no deformation", "wedge-shaped vertebrae", "inverse wedge-shaped vertebrae", "flat vertebrae" and "fish vertebrae", are shown in Fig. 2.

In the judgment as described above, explanation has been made by selecting the upper limit as 1.4 and the lower limit as 0.7 as the judgment standard for c/d as a preferable example, but these values can be selected freely, for example, in the case of the upper limit, from the range of 1.25 to 1.55, preferably from 1.33 to 1.5, more preferably from 1.4 to 1.45. The lower limit can be selected freely from the range of 0.8 to 0.6, preferably from 0.75 to 0.65.

As the judgment standard for a/c and a/d, 0.8 was selected in the case of "no deformation" and "fish vertebrae", but this value can be freely selected from the range of 0.65 to 0.9, preferably from 0.7 to 0.85, more preferably from 0.75 to 0.8.

As the judgment standard for c and d, $\bar{c} - 2\sigma_c$ and $\bar{d} - 1.5\sigma_d$ were selected in the case of "no deformation", "flat vertebrae", and "fish vertebrae" but the judgment standard for c can be freely selected from the range of $\bar{c} - 1.0\sigma_c$ to $\bar{c} - 2.5\sigma_c$, preferably $\bar{c} - 1.25\sigma_c$ to $\bar{c} - 2.25\sigma_c$, more preferably $\bar{c} - 1.5\sigma_c$ to $\bar{c} - 2.0\sigma_c$. The judgment standard for d also can be similarly selected from the range of $\bar{d} - 1.0\sigma_d$ to $\bar{d} - 2.5\sigma_d$,

preferably $\overline{d}$ - $1.25\sigma_d$ to $\overline{d}$ - $2.25\sigma_d$, more preferably $\overline{d}$ - $1.5\sigma_d$ to $\overline{d}$ - $2.0\sigma_d$.

As mentioned above, although the coefficients $\beta_1$ to $\beta_6$ can be previously set in the present invention, $\beta_1$ to $\beta_6$ may optionally be determined from a discriminating function capable of discriminating two groups with two variants or two groups with one variant by using values obtained by measuring two of a, b, c, d, and e with respect to at least five profile X-ray image in each of two types among four types (i.e., wedge-shaped vertabrae, fish vertabrae flat vertebral, and no deformation). As typical axamples, $\beta_1$ is $z_5$ obtained by the following discriminant function using the ratio c/d derived from c and d, which are obtained by measuring at least 5 profile X-ray images in each of two types selected from "wedge-shaped vertebrae" and "no deformation":

(i) When the standard deviation $\sigma_1$ of c/d of at least 5 profileX-ray image of "wedge-type vertebrae" is substantially the same asthe standard deviation $\sigma_2$ of c/d of at least 5 profile X-ray images of "no deformation".

$$z_5 = \overline{\mu} + \frac{\sigma^2}{\mu_1 - \mu_2} \ell_n \frac{\pi_2}{\pi_1}$$

wherein $\mu_1$ and $\mu_2$ are averages of c/d relating to the profile X-ray images of "wedge-shaped vertebrae" and "no deformation", respectively, $\pi_1$ and $\pi_2$ are numbers of the X-ray images of "wedge-type vertebrae" and "no deformation", respectively, $\overline{\mu}$ is an average of $\overline{\mu}_1$ and $\overline{\mu}_2$, and $\sigma$ is an average of $\sigma_1$ and $\sigma_2$; and

(ii) When $\sigma_1$ and $\sigma_2$ are substantially different,

$$z_5 = \frac{-B \pm \sqrt{B^2 - 4A \cdot C}}{2A}$$

$$A = \frac{1}{\sigma_2^2} - \frac{1}{\sigma_1^2}$$

$$B = -2\frac{\mu_2}{\sigma_2^2} - \frac{\mu_1}{\sigma_1^2}$$

$$C = \frac{\mu_2^2}{\sigma_2^2} - \frac{\mu_1^2}{\sigma_1^2} - 2 \cdot \log \frac{\pi_2}{\pi_1}$$

wherein $\mu_1$ and $\mu_2$ are average of c/d relating to the profile X-ray images of "wedge-shaped vertebrae" and "no deformation", respectively, $\pi_1$ and $\pi_2$ are numbers of the X-ray images of "wedge-type vertebrae" and "no deformation", respectively.

Furthermore, the present inventors have made an intensive study of a method of objective evaluation of vertebral body deformation, and found that the presence of a vertebral body deformation as well as the deformation type can be judged objectively by use of a discriminant function by accurately measuring the lengths of, for example, the front brim (d), rear brim (c), central portion (a), and the vertebral body width (b) from a profile X-ray image of a vertebral body, and further found that the progress over a period of time of the vertebral body deformation can be judged from the change in the type of said deformation as well as the change in the lengths of the vertebral body, and thus accomplished the present invention.

In the prior art, vertebral body deformation has been judged by a physician by visual observation of the profile X-ray image of a vertebral body, but according to the method of the present invention, the lengths of the respective portions of a vertebral body are measured and for the vertebral bodies judged by a physician to be "no deformation", "wedge-shaped vertebrae", "fish vertebrae", and "flat vertebrae", discrimination is made by discriminant functions to determine discriminant formulae for the respective types of vertebral body deformations, and thereafter, the presence of vertebral body deformation and the type of deformation are objectively judged by this discriminant formulae for a patient whose vertebral body deformation is to be judged.

The types of deformed vertebral bodies are classified in the present invention generally into wedge-shaped vertebrae, fish vertebrae, and flat vertebrae.

In the wedge-shaped vertebrae (type I), deformation has occurred at the front brim portion as shown in Fig. 4, and the front brim length has become particularly smaller than the rear brim length, including in addition to the so called wedge-shaped vertebrae deformation with a shortened front brim length like wedge-shaped vertebrae as the result of upper brim pressure fracture, an upper brim depressed fracture, lower brim pressure fracture, and lower brim depressed fracture, etc. In the fish vertebrae (type II), the depressed fracture or pressure fracture has occurred at the central portion, whereby the central length has become particularly smaller than the front brim length and rear brim length (see Fig. 4), including in addition to the so called fish vertebrae deformations with a shortened center length like fish vertebrae as the result of upper brim pressure fracture, an upper brim depressed fracture, lower brim pressure fracture, and lower brim depressed fracture, etc.

In the flat vertebrae (type III), the front brim portion, central portion, and rear brim portion are deformed relatively uniformly under pressure (see Fig. 4); namely, all of the front rear length, central length, and rear brim length are smaller.

In addition, there is the inverse wedge-shaped vertebrae (type IV). This type is defined as a vertebral body wherein deformation has occurred at the rear brim portion, and thus the rear brim length is smaller than the front brim length, contrary to the wedge-shaped vertebrae where deformation has occurred at the front brim portion, and the front brim portion is smaller than the rear brim length (see Fig. 4). In practice, however, such a vertebrae does not substantially exist. Accordingly, it is sufficient if the four types, wedge-shaped vertebrae, fish vertebrae, flat vertebrae and "no deformation", can be classified.

The judgment method of the second aspect of the present invention is now described in more detail.

(i) First, the vertebral body, central length (a), vertebral body width (b), rear brim length (c), and front brim length (d) of each of the vertebrae judged by a physician as "no deformation", wedge-shaped vertebrae, fish vertebrae and flat vertebrae, are measured from a profile X-ray of the vertebral body.

The above measurement is used to determine the discriminant formula for discriminating the presence of vertebral body deformation as well as the deformation type. Accordingly, the vertebral bodies of these respective types should be preferably vertebral bodies of persons generally 50 to 75 years old, in view of the age of the patient whose vertebral body deformation is to be judged. Also, it is preferable to perform measurements separately for men and women, and it is desirable to perform measurements of about 50 or more vertebral bodies for both men and women. However, since wedge-shaped, vertebrae, fish vertebrae and flat vertebrae do not widely occur, the discriminant formula for discrimination of the vertebral body deformation type according to the method of the present invention can be determined if at least 5 each of these deformed vertebral bodies can be measured.

Also, from the first to twelfth thoracic vertebrae and the first to the fifth lumbar vertebrae, the size differs for each vertebral body, and therefore, it is necessary to measure each vertebral body and determine each deformation type.

When making a profile X-ray image of a vertebral body, the thoracic vertebrae from the first thoracic vertebrae to the twelfth thoracic vertebrae, lumbar vertebrae from the first lumbar vertebrae to the fifth lumbar vertebrae should be included, giving a total of 17 vertebral bodies, and therefore, for example, it is advantageous to take the photograph of the profile X-ray image with the eighth thoracic vertebrae as the center separately from the profile X-ray image with the third lumbar vertebrae as the center. In the profile X-ray image with the eighth thoracic vertebrae as the center, the first thoracic vertebrae cannot be accurately measured in most cases, and further, the second and the third thoracic vertebrae are unclear in many cases. However, since the frequency of vertebral body deformation is not great in these vertebral bodies, it is sufficient if measurement can be made from the fourth thoracic vertebrae or the fifth thoracic vertebrae.

The center length (a), width (b), rear brim length (c), and front brim length (d) of a vertebral body are specifically as shown in Fig. 4. For determination of these values, from the profile X-ray image of a vertebral body, the central length (a), width (b), rear brim length (c), and front brim length (d) may be measured for each vertebral body with a scale or slide calipers, but the eight points of 1 to 8 shown in Fig. 4 can be input through a digitizer into a computer to measure the central length (a) (3-4), width (b) (7-8), rear brim length (c) (5-6), and front brim length (d) (1-2). Also, for example, the degree of blackening may be recorded with a TV camera, etc., and the lengths of the respective portions of the vertebral body may be automatically measured by image processing.

(ii) Next, discriminant formula for discriminating "no deformation", wedge-shaped vertebrae, fish vertebrae, and flat vertebrae is determined. Such a discriminating analysis is described in, for example, Chuichi Okuno et al "Multivariate Analytical Method" (published by Nikkagiren); Chuichi Okuno et al "Overall Multivariate Analytical Method" (published by Nikkagiren); Koichi Sugiyama "Introduction to Multivariate Data Analysis" (published by Asakura Shoten), and others, and calculation can be made by referring to these literatures. However, by inputting the central length (a), vertebral body width (b), rear brim length (c), and front brim length (d) of the respective deformation types determined in (i) by a commercially available computer program (e.g., BMD multivariate analysis program from IBM Co.), the discriminant formula for discriminating "no deformation", wedge-shaped vertebrae, fish vertebrae, and flat vertebrae can be simply obtained. Such discriment formulae are specifically described as follows. That is, the following formulae and the vertebral body is judged from the maximum value of $z_1$ to $z_4$:

$$z_1 = a_{10} + a_{11}x_1 + a_{12}x_2 + a_{13}x_3 + a_{14}x_4$$
$$z_2 = a_{20} + a_{21}x_1 + a_{22}x_2 + a_{23}x_3 + a_{24}x_4$$
$$z_3 = a_{30} + a_{31}x_1 + a_{32}x_2 + a_{33}x_3 + a_{34}x_4$$
$$z_4 = a_{40} + a_{41}x_1 + a_{42}x_2 + a_{43}x_3 + a_{44}x_4$$

wherein $z_1$, $z_2$, $z_3$, and $z_4$ are discriminant values of "wedge-shaped vertebrae", "fish vertebrae", "flat vertebrae", and "no deformation", respectively, $x_1$, $x_2$, $x_3$, and $x_4$ are variables corresponding to a central length, vertebral body width, rear brim length, and front brim length, respectively, $a_{10}$, $a_{20}$, $a_{30}$, and $a_{40}$ are constants, and $a_{11}$ to $a_{44}$ are coefficients. This discriminant formula must be determined for each vertebral body from the first thoracic vertebrae to the fifth lumbar vertebrae, generally from the fourth thoracic vertebrae to the fifth lumbar vertebrae.

(iii) Next, from the profile X-ray image of the vertebral body of the patient whose vertebral body deformation is to be judged, the central length (a), vertebral body width (b), rear brim length (c), and front brim length (d) are measured for each vertebral body as described in (i), and by substituting a, b, c and d in the discriminant formula determined in (ii), the discriminant values for "no deformation", wedge-shape vertebrae, fish vertebrae, and flat vertebrae are determined respectively, and the type which has the highest numerical value is judged to be the vertebral body deformation type. For example, when the vertebral body deformation type of the third lumbar vertebrae of the patient is to be judged, the respective measured values of a, b, c, and d are substituted in the discriminant formula of the third lumbar vertebrae determined in (ii) as a matter of course. Having described above the method for judging, the present invention is not limited to the above classification of the four vertebral deformation types of "no deformation", wedge-shaped vertebrae, fish vertebrae and flat vertebrae from the four measured values of central length (a), vertebral body width (b), rear brim length (c), and front brim length (d), but it is also possible to classify the vertebral body deformation types by use of the discriminant function as described below.

(A) Method for classifying the three vertebral body deformation types from three or four measured values.

For example, in the first to the eleventh thoracic vertebrae, when fish vertebrae does not substantially exist, the case occurs wherein classification is required for only the three types of "no deformation", wedge-shaped vertebrae, and flat vertebrae, or the case when sufficient measured values of a, b, c, and d of fish vertebrae cannot be obtained for determining the discriminant formula even if a classification of the four types including fish vertebrae is desired.

In such a case, it is possible to determine the discriminant formula for classifying the three types of "no deformation", wedge-shaped vertebrae, and flat vertebrae from the four measured values of the central length (a), vertebral body width (b), rear brim length (c), and front brim length (d), or from three of these four measured values, for example, three measured values of a, c, and d.

(B) Method for classifying two vertebral body deformation types from two to four measured values.

Fish vertebrae and flat vertebrae can be judged relatively easily even when visually observed, and rarely occur, and therefore, in some cases they are difficult to judge with the use of a discriminant formula. Accordingly, when it is desired to classify only "no deformation" and wedge-shaped vertebrae by use of a discriminant formula, three, for example, a, c and d, or two, for example, measured values of c and d, can be used to determine the discriminant formula.

Also, in such a case, it is possible to determine the discriminant value for classifying "no deformation" and wedge-shaped vertebrae by using of a single variate discriminant function of c/d.

Specifically speaking, the discriminant function is represented by the following formula in which the ratio (c/d) of c and d measured from at least 5 profile X-ray images in each of two types of :wedge-shaped vertebrae" and "no deformation" and the ratio (c/d) of less than $z_5$ obtained from the vertebral body to be judged is judged as "no deformation" and the ratio (c/d) equal to or larger than $z_5$ is judged as "wedge-

EP 0 245 098 B1

shaped vertebrae".

(i) When the standard deviation $\sigma_1$ of c/d of at least 5 profile X-ray image of "wedge-type vertebrae" is substantially the same asthe standard deviation $\sigma_2$ of c/d of at least 5 profile X-ray images of "no deformation".

$$z_5 = \bar{\mu} + \frac{\sigma^2}{\mu_1 - \mu_2} \ell_n \frac{\pi_2}{\pi_1}$$

wherein $\mu_1$ and $\mu_2$ are averages of c/d relating to the profile X-ray images of "wedge-shaped vertebrae" and "no deformation", respectively, $\pi_1$ and $\pi_2$ are numbers of the X-ray images of "wedge-type vertebrae" and "no deformation", respectively, $\bar{\mu}$ is an average of $\bar{\mu}_1$ and $\bar{\mu}_2$, and $\sigma$ is an average of $\sigma_1$ and $\sigma_2$ ; and

(ii) When $\sigma_1$ and $\sigma_2$ are substantially different,

$$z_5 = \frac{-B \pm \sqrt{B^2 - 4 A \cdot C}}{2A}$$

$$A = \frac{1}{\sigma_2^2} - \frac{1}{\sigma_1^2}$$

$$B = -2 \frac{\mu_2}{\sigma_2^2} - \frac{\mu_1}{\sigma_1^2}$$

$$C = \frac{\mu_2^2}{\sigma_2^2} - \frac{\mu_1^2}{\sigma_1^2} - 2 \cdot \log \frac{\pi_2}{\pi_1}$$

wherein $\mu_1$ and $\mu_2$ are averages of c/d relating to the profile X-ray images of "wedge-shaped vertebrae" and "no deformation", respectively, $\pi_1$ and $\pi_2$ are numbers of the X-ray images of "wedge-type vertebrae" and "no deformation", respectively.

The apparatus for judging the deformation of vertebral body according to the present invention is used for the practice of the above-mentioned method of the present invention. This apparatus is basically composed of an imputting means for at least two indices of a, b, c, and d, an arithmetic means for effecting necessary operations for the descrimination, a discriminating means by using the calculation results, a means for inputting the discriminant function and/or standard necessary for the calculation and discrimination, and an output means for outputting the judgment result.

As the inputting means, a rule means, a digitizer means, an image processing means in which an image inputting means such as a TV camera is used.

In the preferred embodiment of the present apparatus, c/d, a/c, and a/d are determined by the arithmetic means, and $\bar{c}$, $\bar{d}$, $\sigma_c$, $\sigma_d$, and $\beta_1 - \beta_6$ are inputted as discriminating standards by the discriminating standard inputting means, and the discriminating means can discriminate the results by these standards. Furthermore, the present apparatus can be further provided with a means for obtained the discriminating standards from a number of the profile X-ray images having no substantial deformation. Furthermore, the present apparatus can be optionally provided with an arithmetic means capable of discriminating two groups with two variants, which are used for determining the indices $\beta_1$ to $\beta_6$, or of discriminating two groups with one variant. In this case, the data of at least two types of the four types of the vertebral body, i.e., "wedge-shaped vertebrae", "fish vertebrae", "flat vertebrae", and "no deformation" should be input.

I another preferred embodiment of the present apparatus, the arithmetic means is connected to a discriminating standard input means for inputting the discriminant function, whereby the arithmetic means calculates the discriminating values with said discriminant function and the discriminating means determine

9

the type of the vertebral body based on the maximum discriminating value. In this case, the present apparatus is provided with a function capable of discriminating the four types of the vertebral body, "wedge-shaped vertebrae", "fish vertebrae", "flat vertebrae", and "no deformation" from a one-dimensional formula containing four variables a, b, c, and d. Furthermore, there is also the apparatus which is capable of judging the two types of the vertebral body with one variant.

According to the first aspect of the judgment method and the apparatus of the present invention, the type of vertebral body deformation can be objectively evaluated, and also the change in the said deformation type and the progress over a period of time of the vertebral body deformation can be judged.

Also, the present method and apparatus is very useful for determining the progress of bone disease such as osteoporosis, etc., and for confirmation of the effect of therapy.

According to the second aspect of the judgment method and apparatus of the present invention, the type of vertebral body deformation can be objectively evaluated, and further the change in the type of deformation type and the progress over a period of time of the vertebral body deformation can be judged.

Also, the present method and apparatus is very useful for determining the progress of bone disease such as osteoporosis, etc., and for confirmation of the effect of therapy.

Examples

The present invention will now be further illustrated by, but is by no means limited to, the following Examples.

Example 1

From the profile X-ray images of thoracic and lumbar vertebrae, with the eighth thoracic vertebrae and the third lumbar vertebrae as the center, of 50 to 75 years old women, the front brim length (d), central length (a), rear brim length (c), and vertebral body width (b) were measured for each vertebral body, and for the vertebral bodies classified by a physician as "no deformation", the average values ($\bar{x}$) and standard deviations ($\sigma$) for the respective vertebral bodies of the measured values of a, b, c, and d, and the calculated values of c/d, are as shown in Table 1.

Table 1

Unit: mm

| Site | | Data number | a | | b | | c | | d | | c/d | |
|---|---|---|---|---|---|---|---|---|---|---|---|---|
| | | | $\bar{x}$ | σ | $\bar{x}$ | σ | $\bar{x}$ | σ | $\bar{x}$ | σ | $\bar{x}$ | σ |
| 2nd | thoracic vertebrae | 11 | 18.27 | 3.02 | 25.40 | 4.03 | 21.60 | 3.80 | 18.69 | 2.97 | 1.15 | 0.11 |
| 3rd | " | 56 | 17.08 | 2.20 | 25.30 | 2.67 | 21.03 | 2.44 | 18.46 | 2.10 | 1.14 | 0.11 |
| 4th | " | 141 | 17.83 | 2.25 | 26.65 | 2.60 | 21.28 | 2.17 | 18.41 | 2.07 | 1.16 | 0.10 |
| 5th | " | 194 | 18.20 | 2.12 | 27.22 | 2.52 | 21.74 | 2.21 | 18.62 | 2.05 | 1.17 | 0.09 |
| 6th | " | 210 | 18.87 | 1.90 | 28.31 | 2.70 | 22.02 | 1.81 | 18.67 | 2.02 | 1.18 | 0.11 |
| 7th | " | 210 | 19.28 | 1.77 | 29.57 | 2.64 | 22.49 | 1.95 | 18.99 | 2.12 | 1.19 | 0.11 |
| 8th | " | 207 | 19.78 | 1.69 | 30.79 | 2.75 | 22.92 | 1.88 | 19.74 | 2.05 | 1.16 | 0.13 |
| 9th | " | 220 | 20.07 | 1.99 | 31.78 | 2.95 | 23.30 | 1.88 | 20.52 | 2.19 | 1.14 | 0.10 |
| 10th | " | 229 | 21.03 | 2.07 | 32.89 | 3.19 | 24.32 | 2.21 | 21.44 | 2.22 | 1.14 | 0.10 |
| 11th | " | 214 | 22.04 | 2.42 | 33.83 | 3.06 | 26.11 | 2.57 | 22.25 | 2.37 | 1.18 | 0.11 |
| 12th | " | 194 | 24.16 | 2.22 | 34.52 | 3.26 | 28.20 | 3.00 | 24.52 | 2.45 | 1.15 | 0.11 |
| 1st | lumbar vertebrae | 215 | 26.18 | 2.34 | 34.98 | 2.94 | 30.32 | 2.68 | 26.72 | 2.36 | 1.14 | 0.09 |
| 2nd | " | 233 | 26.57 | 2.76 | 36.09 | 3.31 | 30.88 | 2.72 | 27.55 | 2.83 | 1.13 | 0.11 |
| 3rd | " | 237 | 27.10 | 2.78 | 37.44 | 3.21 | 30.58 | 2.76 | 28.62 | 3.14 | 1.07 | 0.11 |
| 4th | " | 251 | 26.68 | 3.11 | 37.69 | 3.02 | 28.07 | 2.72 | 28.08 | 3.27 | 1.00 | 0.09 |
| 5th | " | 225 | 25.91 | 2.85 | 37.72 | 3.05 | 26.34 | 2.54 | 27.83 | 3.37 | 0.95 | 0.12 |

Example 2

From the profile X-ray image of the thoracic and lumber vertebrae of a 70 years old osteoporosis patient (woman), the center length (a), vertebral body width (b), rear brim length (c), and front brim length (d) were measured for each vertebral body, and c/d, a/c and a/d were calculated. The results of the judgment of the presence of deformation of each vertebral body, and the type of change, are shown in Table 2. The judgment was made according to the judgment standard shown in Table 2. The same standards were also used in Examples 3 and 4.

Table 2

| Measurement site: Site: | a | b | c | d | c/d | a/c | a/d | Presence of deformation / Type of deformation |
|---|---|---|---|---|---|---|---|---|
| 5th thoracic vertebrae | 13.5 | 28.8 | 20.6 | 15.9 | 1.30 | 0.65 | 0.85 | No deformation |
| 6th " | 13.1 | 33.3 | 22.0 | 13.5 | 1.63 | 0.60 | 0.97 | Type I |
| 7th " | 13.3 | 36.5 | 21.3 | 17.1 | 1.25 | 0.63 | 0.78 | Type II |
| 8th " | 12.9 | 38.2 | 21.8 | 13.2 | 1.64 | 0.59 | 0.97 | Type I |
| 9th " | 21.3 | 35.9 | 23.8 | 22.5 | 1.05 | 0.90 | 0.95 | No deformation |
| 10th " | 17.8 | 38.5 | 23.8 | 21.8 | 1.09 | 0.75 | 0.82 | " |
| 11th " | 15.2 | 38.2 | 25.8 | 16.8 | 1.53 | 0.59 | 0.91 | Type I |
| 12th " | 20.1 | 38.7 | 29.1 | 16.9 | 1.73 | 0.69 | 1.19 | Type I |
| 1st lumbar vertebrae | 12.7 | 40.4 | 27.3 | 17.8 | 1.53 | 0.46 | 0.71 | Type I |
| 2nd " | 18.7 | 40.8 | 27.9 | 22.0 | 1.27 | 0.67 | 0.85 | No deformation |
| 3rd " | 13.7 | 45.4 | 28.1 | 25.2 | 1.11 | 0.49 | 0.54 | Type II |
| 4th " | 17.2 | 42.4 | 26.5 | 31.2 | 0.85 | 0.65 | 0.55 | Type II |
| 5th " | 21.6 | 42.5 | 23.7 | 23.8 | 1.00 | 0.91 | 0.91 | No deformation |

Example 3

From the profile X-ray image, with the third lumbar vertebrae as the center, of ten osteoporosis patients (women) 59 to 76 years old, the central length (a), vertebral body width (b), rear brim length (c), and front brim length (d) of the third lumber vertebrae were measured and c/d, a/c, and a/d were calculated. The results of the judgment of the presence of deformation, and the type of deformation, are shown in Table 3.

Table 3

| Measurement site: Case (age): | a | b | c | d | c/d | a/c | a/d | Presence of deformation / Type of deformation |
|---|---|---|---|---|---|---|---|---|
| 1 (59) | 31.4 | 38.6 | 38.0 | 34.2 | 1.11 | 0.83 | 0.92 | No deformation |
| 2 (63) | 28.0 | 40.2 | 33.3 | 32.4 | 1.03 | 0.84 | 0.86 | " |
| 3 (64) | 26.9 | 41.1 | 30.1 | 28.4 | 1.06 | 0.90 | 0.95 | " |
| 4 (66) | 18.3 | 41.0 | 31.6 | 21.4 | 1.47 | 0.58 | 0.85 | Type I |
| 5 (76) | 20.8 | 34.7 | 26.3 | 16.6 | 1.59 | 0.79 | 1.25 | " |
| 6 (65) | 21.2 | 37.6 | 32.7 | 17.4 | 1.88 | 0.65 | 1.22 | " |
| 7 (68) | 16.6 | 40.7 | 25.6 | 24.8 | 1.03 | 0.65 | 0.67 | Type II |
| 8 (66) | 20.4 | 41.4 | 29.6 | 30.9 | 0.96 | 0.69 | 0.66 | " |
| 9 (72) | 14.2 | 33.8 | 22.4 | 16.7 | 1.34 | 0.63 | 0.85 | Type III |
| 10 (62) | 21.2 | 31.9 | 23.8 | 21.7 | 1.09 | 0.89 | 0.97 | " |

Example 4

From the profile X-ray image, with the eighth thoracic vertebrae and the third lumbar vertebrae as the center, of a 67 years old osteoporosis patient (woman), the center length (a), vertebral body width (b), rear brim length (c), and front brim length (d) from the sixth thoracic vertebrae to the fifth lumbar vertebrae were measured, and c/d, a/c, and a/d were calculated to judge the presence of deformation and the type of deformation. Six months later, the profile X-ray image of the same site was photographed again to judge the presence of deformation and the type of deformation according to the same method, and at the same time, the change in the ratios of a, b, c, and d were calculated. The results are shown in Table 4.

Table 4

| | At initiation of experiment | | | | | | | | After 6 months | | | | | | | | Change in ratio (%) | | | |
|---|---|---|---|---|---|---|---|---|---|---|---|---|---|---|---|---|---|---|---|---|
| | a | b | c | d | c/d | a/c | a/d | Type of deformation | a | b | c | d | c/d | a/c | a/d | Type of deformation | a | b | c | d |
| 6th thoracic vertebrae | 15.8 | 30.8 | 23.2 | 15.9 | 1.46 | 0.68 | 0.99 | Type I | 16.7 | 29.0 | 22.4 | 15.0 | 1.49 | 0.74 | 1.11 | Type I | 105 | 94 | 96 | 94 |
| 7th | 19.6 | 32.1 | 26.0 | 19.5 | 1.33 | 0.75 | 1.01 | No deformation | 19.6 | 33.8 | 22.3 | 17.3 | 1.29 | 0.88 | 1.14 | No deformation | 100 | 105 | 85 | 88 |
| 8th | 16.1 | 31.7 | 22.1 | 16.5 | 1.37 | 0.73 | 0.98 | " | 14.7 | 32.7 | 21.9 | 16.3 | 1.34 | 0.67 | 0.90 | " | 91 | 103 | 99 | 98 |
| 9th | 21.6 | 35.2 | 26.5 | 23.6 | 1.12 | 0.82 | 0.92 | " | 19.9 | 36.0 | 24.9 | 22.2 | 1.12 | 0.80 | 0.89 | " | 91 | 102 | 94 | 94 |
| 10th | 16.6 | 38.1 | 23.6 | 14.4 | 1.63 | 0.70 | 1.15 | Type I | 14.0 | 35.2 | 22.9 | 14.5 | 1.58 | 0.61 | 0.97 | Type I | 84 | 92 | 97 | 100 |
| 11th | 24.2 | 37.8 | 29.1 | 25.4 | 1.15 | 0.83 | 0.95 | No deformation | 24.1 | 36.0 | 28.6 | 25.0 | 1.14 | 0.84 | 0.96 | No deformation | 99 | 95 | 98 | 98 |
| 12th | 26.3 | 37.8 | 31.3 | 27.5 | 1.14 | 0.84 | 0.96 | " | 25.9 | 39.3 | 30.8 | 26.9 | 1.15 | 0.84 | 0.96 | " | 98 | 103 | 98 | 97 |
| 1st lumbar vertebrae | 27.5 | 35.1 | 33.3 | 28.1 | 1.19 | 0.82 | 0.98 | " | 22.9 | 34.6 | 31.8 | 26.4 | 1.21 | 0.72 | 0.87 | " | 83 | 98 | 95 | 94 |
| 2nd | 24.0 | 38.7 | 30.4 | 26.8 | 1.14 | 0.79 | 0.89 | " | 20.0 | 35.6 | 23.6 | 20.8 | 1.13 | 0.85 | 0.96 | Type III | 83 | 92 | 77 | 77 |
| 3rd | 14.3 | 41.6 | 21.4 | 20.7 | 1.03 | 0.67 | 0.69 | Type III | 9.1 | 39.8 | 21.1 | 20.3 | 0.87 | 0.43 | 0.44 | " | 63 | 95 | 98 | 98 |
| 4th | 19.0 | 40.0 | 29.1 | 22.1 | 1.32 | 0.65 | 0.86 | No deformation | 19.2 | 36.4 | 27.2 | 22.6 | 1.20 | 0.71 | 0.85 | No deformation | 101 | 91 | 93 | 102 |
| 5th | 19.3 | 38.4 | 26.4 | 26.3 | 1.00 | 0.73 | 0.73 | Type II | 18.6 | 38.4 | 26.4 | 25.4 | 1.04 | 0.70 | 0.73 | Type II | 96 | 99 | 100 | 96 |

14

The second lumbar vertebrae changed from a "no deformation" classification to a type III (flat vertebrae) within six months.

Although not showing a clear deformation, the conditions of the front brim and rear brim of the seventh thoracic vertebrae, and the center of the tenth thoracic vertebrae, tend to have worsened. Also, the conditions of the centers of the tenth thoracic vertebrae and the third lumbar vertebrae had further worsened.

On the other hand, substantially no change has occurred in the sixth, eighth, ninth, eleventh, and twelfth thoracic vertebrae, or in the fourth and fifth lumbar vertebrae.

Example 5

For 186 vertebral bodies, including 6 vertebral bodies judged as wedge-shaped vertebrae, 25 vertebral bodies judged as fish vertebrae, eight vertebral bodies judged as flat vertebrae and 147 vertebral bodies judged as "no deformation" according to visual observation by a physician of a profile X-ray image, with the third lumbar vertebrae as the center, of osteoporosis patients (women), eight points of 1 to 8 shown in Fig. 4 were input through a digitizer into a computer to measure the central length (a), vertebral body width (b), rear brim length (c), and front brim length (d), and calculation was performed by a computer by inputting the above a, b, c, and d into a multivariate discriminant analytical program in the BMD multivariate analytical program of IBM Co. to obtain the coefficients and constants of the discriminant formula as shown in Table 5.

## Table 5

| FUNCTION | 1 (wedge-shaped vertebrae) | 2 (fish vertebrae) | 3 (flat vertebrae) | 4 (no defor-mation) |
|---|---|---|---|---|
| COEFFICIENT 1 (a) | 1.41660 | 0.14656 | −0.19733 | 1.94241 |
| 2 (b) | 3.31142 | 3.63664 | 3.37930 | 3.51934 |
| 3 (c) | 2.39311 | 1.77030 | 1.42894 | 1.43377 |
| 4 (d) | −0.73812 | 1.23660 | 1.18794 | 0.60223 |
| CONSTANT | −100.52945 | −114.00328 | −89.05205 | −123.11888 |

The discriminant value for each vertebral body was calculated by substituting the measured values of a, b, c, and d, into the above four discriminant formulae, and the type having the maximum numerical value was judged to be the vertebral body deformation type.

The coincidence of the vertebral body deformation type thus judged according to the discriminant formula with the vertebral body deformation type according to the physician's judgment is shown in Table 6, indicating a good coincidence between both judgments. The coincidence ratio of the respective types is 94.6%.

Table 6

| Discriminant analysis: | Wedge-shaped vertebrae | Fish vertebrae | Flat vertebrae | No deformation | Total | Coincidence ratio (%) |
|---|---|---|---|---|---|---|
| Physician's judgments: | | | | | | |
| Wedge-shaped vertebrae | (6) | 0 | 0 | 0 | 6 | 100.0 |
| Fish vertebrae | 0 | (23) | 2 | 0 | 25 | 92.0 |
| Flat vertebrae | 0 | 1 | (7) | 0 | 8 | 87.2 |
| No deformation | 2 | 1 | 0 | (140) | 147 | 95.2 |

The mark ○ shows the number of vertebral bodies for which both judgments coincide.

Example 6

From a profile X-ray image, with the third lumbar vertebrae as the center, of 10 osteoporosis patients (women), the central length (a), vertebral body width (b), rear brim length (c), and front brim length (d) of the third lumbar vertebrae were measured and substituted in the discriminant formula determined in Example 5 (coefficients and constants are shown in Table 5) to calculate the respective discriminant values, and the type having the maximum numerical value was judged to be the vertebral body deformation type. The results are shown in Table 7.

16

Table 7

| No. | Vertebral body measured values (mm) | | | | Discriminant values | | | | Deformation type |
|---|---|---|---|---|---|---|---|---|---|
| | a | b | c | d | Wedge-shaped vertebrae | Fish vertebrae | Flat vertebrae | No deformation | |
| 1 | 27.0 | 37.8 | 31.6 | 27.3 | 118.36 | 117.12 | 110.94 | <u>124.11</u> | No deformation |
| 2 | 27.5 | 33.4 | 32.6 | 28.1 | 106.30 | 103.95 | 98.35 | <u>115.51</u> | No deformation |
| 3 | 15.9 | 40.2 | 26.0 | 17.0 | <u>104.79</u> | 101.57 | 101.01 | 96.76 | Wedge-shaped vertebrae |
| 4 | 16.9 | 35.2 | 28.2 | 15.6 | <u>95.94</u> | 85.70 | 85.39 | 83.42 | Wedge-shaped vertebrae |
| 5 | 20.5 | 40.3 | 30.5 | 20.0 | <u>120.19</u> | 114.28 | 110.43 | 114.30 | Wedge-shaped vertebrae |
| 6 | 19.6 | 38.6 | 28.1 | 26.9 | 102.45 | <u>112.25</u> | 109.63 | 107.29 | Fish vertebrae |
| 7 | 20.8 | 36.9 | 28.7 | 30.0 | 97.67 | <u>111.14</u> | 108.19 | 106.36 | Fish vertebrae |
| 8 | 21.6 | 32.6 | 30.8 | 30.8 | 89.00 | <u>100.33</u> | 97.45 | 96.28 | Fish vertebrae |
| 9 | 13.2 | 34.0 | 21.3 | 17.3 | 68.96 | 70.68 | <u>74.23</u> | 63.14 | Flat vertebrae |
| 10 | 18.8 | 31.0 | 21.8 | 21.5 | 65.06 | 66.67 | <u>68.69</u> | 66.70 | Flat vertebrae |

Example 7

From vertebral body profile X-ray images with the eighth thoracic vertebrae and the third thoracic vertebrae as the centers thereof, respectively, according to a physician's judgment, as shown in Table 8, from the sixth thoracic vertebrae to the third thoracic vertebrae, 257 vertebral bodies of wedge-shaped vertebrae and 2,157 vertebral bodies of "no deformation" were selected, and the central length (a), vertebral width (b), rear brim length (c), and front brim length (d) of each vertebral body were measured as in

17

Example 5, and in addition, the c/d was calculated.

For discriminating wedge-shaped vertebrae from "no deformation" with one variate of c/d, the value of c/d for each vertebral body was input to the one variate discriminant analytical program in the BMD multivariate analytical program of IBM Co. to obtain the discriminant values shown in Table 8. The coincidence with the physicians judgment when discrimination was made between wedge-shaped vertebrae- and "no deformation" by use of these discriminant values is shown in Table 8. For all vertebral bodies, the coincidence ratio of "no deformation" was 98.15%, and the coincidence ratio of wedge-shaped vertebrae 87.16%, thus giving good results.

Table 8

| Site | Number of vertebral bodies used for discrimination | | Discriminant c/d value according to discriminant function | Coincidence with physician's judgment | | | |
|---|---|---|---|---|---|---|---|
| | Wedge-shaped vertebrae | No deformation | | Wedge-shaped vertebrae | | No deformation | |
| | | | | Coincidence number | Coincidence ratio (%) | Coincidence number | Coincidence ratio (%) |
| Th 6 | 14 | 209 | 1.456 | 11 | 78.57 | 208 | 99.52 |
| " 7 | 22 | 205 | 1.422 | 18 | 81.82 | 204 | 99.51 |
| " 8 | 25 | 206 | 1.399 | 21 | 84.00 | 204 | 99.03 |
| " 9 | 19 | 217 | 1.388 | 18 | 94.74 | 212 | 97.70 |
| " 10 | 9 | 228 | 1.420 | 6 | 66.67 | 226 | 99.12 |
| " 11 | 30 | 214 | 1.422 | 22 | 73.33 | 207 | 96.73 |
| " 12 | 55 | 193 | 1.363 | 51 | 92.73 | 186 | 96.37 |
| L 1 | 52 | 214 | 1.341 | 49 | 94.23 | 212 | 99.07 |
| " 2 | 25 | 238 | 1.406 | 23 | 92.00 | 229 | 96.22 |
| " 3 | 6 | 233 | 1.407 | 5 | 83.33 | 229 | 98.38 |
| Total/ average | 257 | 2,157 | 1.402 | 224 | 87.16 | 2,117 | 98.15 |

Th: thoracic vertebrae
L: lumbar vertebrae

Example 8

From the profile X-ray images, with the eighth thoracic vertebrae as the center, of 10 osteoporosis patients (women), as described in Example 5, the center length (a), vertebral body width (b), rear brim length (c), and front brim length (d) of the eighth thoracic vertebrae were measured, and in addition, the c/d was calculated. The results are shown in Table 9. Since these vertebral bodies were neither clearly fish vertebrae nor flat vertebrae, a discrimination between wedge-shaped vertebrae and "no deformation" by the c/d determined in Example 7 was conducted. The results are shown in the right hand column in Table 9.

Table 9

| No. | Vertebrae body measured values (mm) | | | | | Deformation type |
|---|---|---|---|---|---|---|
| | a | b | c | d | c/d | |
| 1 | 20.0 | 22.5 | 24.7 | 16.5 | 1.50 | Wedge-shaped vertebrae |
| 2 | 21.6 | 30.2 | 24.4 | 21.3 | 1.14 | No deformation |
| 3 | 17.5 | 32.9 | 21.7 | 13.6 | 1.60 | Wedge-shaped vertebrae |
| 4 | 17.7 | 38.3 | 29.6 | 15.6 | 1.90 | " |
| 5 | 22.0 | 28.8 | 23.8 | 19.2 | 1.24 | No deformation |
| 6 | 12.9 | 38.2 | 21.8 | 13.2 | 1.64 | Wedge-shaped vertebrae |
| 7 | 21.0 | 28.3 | 24.5 | 21.2 | 1.15 | No deformation |
| 8 | 18.9 | 32.0 | 22.4 | 18.5 | 1.21 | " |
| 9 | 19.8 | 30.8 | 24.3 | 18.7 | 1.30 | " |
| 10 | 19.3 | 31.3 | 22.2 | 15.6 | 1.43 | Wedge-shaped vertebrae |

## Claims

1. A method for judging deformation of a vertebral body in a subject, comprising the steps of:

(i) measuring a central length a, a rear brim length c and a front brim length d from a plurality of profile X-ray images obtained from vertebral bodies having substantially no deformation at a portion corresponding to a portion of said vertebral body to be judged, storing the measurements in a memory, and determining values of c/d, a/c and a/d, the average values $\bar{c}$ and $\bar{d}$ of the rear brim lengths c and front brim lengths d, respectively, and the standard deviations $\sigma_c$ and $\sigma_d$ of the values c and d, respectively:

(ii) measuring a, c, and d from a plurality of profile X-ray images obtained from vertebral bodies having at least one deformation, storing the measurements in said memory, and determining values of c/d, a/c, a/d, and $\bar{c}$, $\bar{d}$, $\sigma_c$, and $\sigma_d$;

(iii) preparing a judgement standard from the data obtained in steps (i) and (ii) for determining the nature of the deformity according to a classification consisting of "no deformation" vertebrae, "wedge-shaped" vertebrae, "inverse wedge-shaped" vertebrae, "fish" vertebrae, and "flat" vertebrae;

(iv) measuring a, c, and d from a profile X-ray image of said vertebral body to be judged, storing the measurements in said memory, and determining c/d, a/c, and a/d; and

(v) classifying deformation of the vertebral body to be judged from c/d in the case of "wedge-shaped" and "inverse wedge-shaped" vertebrae, from $\bar{c}$, $\bar{d}$, $\sigma_c$, $\sigma_d$, and c/d in the case of "flat" vertebrae, and from $\bar{c}$, $\bar{d}$, $\sigma_c$, $\sigma_d$, a/c, a/d, and c/d in the case of the "no deformation" and "fish" vertebrae, in order to judge deformation and to monitor the effect of therapy on said vertebral body to be judged.

2. A method according to claim 1, wherein said step (v) is carried out according to the following sub-steps:

(i) c/d $\geq \beta_1$ ... wedge-shaped vertebrae (or anterior wedge fracture)

(ii) c/d $\leq \beta_2$ ... inverse wedge-shaped vertebrae (or inverse anterior wedge fracture)

(iii) c < $\bar{c}$ - $\beta_3\sigma_c$, d < $\bar{d}$ - $\beta_4\sigma_d$, and $\beta_2$ < c/d < $\beta_1$ ... flat vertebrae (or compression fracture)

(iv) $\beta_2$ < c/d < $\beta_1$, a/c $\leq \beta_5$, a/d $\leq \beta_6$ and c $\geq \bar{c}$ - $\beta_3\sigma c$ and/or d $\geq$ d - $\beta_4\sigma_d$ ... fish vertebrae (or central biconcave fracture)

(v) $\beta_2 < c/d$ $\beta_1$, $c \geq \bar{c}$ - $\beta_3\sigma_c$ and/ord $\geq \bar{d}$ - $\beta_4\sigma_d$, and a/c > $\beta_5$ and/or a/d > $\beta_6$ ... No deformation (or no fracture), wherein $\beta_1$ to $\beta_6$ are selected as follows: $1.25 \leq \beta_1 \leq 1.55$, $0.6 \leq \beta_2 \leq 0.8$, $1 \leq \beta_3 \leq 2.5$, $1 \leq \beta_4 \leq 2.5$, $0.65 \leq \beta_5 \leq 0.9$, and $0.65 \leq \beta_6 \leq 0.9$.

3. A method as claimed in claim 2, wherein $\beta_1$ to $\beta_6$ are $1.33 \leq \beta_1 \leq 1.5$, $0.65 \leq \beta_2 \leq 0.75$, $1.25 \leq \beta_3 \leq 2.25$, $1.25 \leq \beta_4 \leq 2.25$, $0.7 \leq \beta_5 \leq 0.85$, and $0.7 \leq \beta_6 \leq 0.85$.

4. A method as claimed in claim 3, wherein $\beta_1$ is 1.4, $\beta_2$ 0.7, $\beta_3$ 2, $\beta_4$ 1.5, $\beta_5$ 0.8, and $\beta_6$ 0.8.

5. A method as claimed in any of claims 2 to 4, wherein at least one of $\beta_1$ to $\beta_6$ are determined by a discriminant function capable of discriminating two groups with one or two variables by using two of a, b, c, and d or the ratio thereof, which are obtained by measuring at least 5 profile X-ray images in each of two types selected from four types, "wedge-shaped vertebrae", "fish vertebrae", "flat vertebrae", and "no deformation".

6. A method as claimed in any preceding claim, wherein the measurement is carried out by a rule means, a digitizer means, or a means for automatically measuring the lengths from image treatment by memorizing the X-ray image with an X-ray cage automatic input means.

7. Apparatus for judging deformation of a vertebral body according to the method of claim 1, the apparatus comprising (i) an input means for inputting values of at least central length a, rear brim length c, and front brim length d from a profile X-ray image of a vertebral body to be judged, (ii) an arithmetic means for performing the calculation of c/d, a/c, a/d, the average values $\bar{c}$, $\bar{d}$ of c and d and the standard deviations $\sigma_c$ and $\sigma_d$ of the values c and d, respectively, necessary for determination of the nature of the deformity according to a classification consisting of "wedge-shaped vertebrae", "inverse wedge-shaped vertebrae", "fish vertebrae", "flat vertebrae", and "no deformation" by using said input values, (iii) a means for discriminating "wedge-shaped" and "inverse wedge-shaped" vertebrae from c/d, "flat" vertebrae from $\bar{c}$, $\bar{d}$, $\sigma_c$, $\sigma_d$, and c/d, and "no deformation" and "fish" vertebrae from $\bar{c}$, $\bar{d}$, $\sigma_c$, $\sigma_d$, a/c, a/d, and c/d, (iv) a means for inputting the judgement standard necessary for the said calculation and discrimination, and (v) an output means for outputting the judgement result.

8. Use of the apparatus according to claim 7, for carrying out the method of any one of claims 2-6.

**Patentansprüche**

1. Verfahren zum Prüfen von Deformationen eines Wirbelkörpers in einem Subjekt, welches die Schritte umfaßt:
   (i) Messen einer mittigen Länge a, einer rückwärtigen Rand-Länge c und einer vorderen Rand-Länge d aus einer Vielzahl von Profil-Röntgenaufnahmen, die von einem Teil von im wesentlichen deformationslosen Wirbelkörpern erhalten wurden, welcher dem Teil des zu beurteilenden Wirbelkörpers entspricht,
   Speichern der Messungen in einem Speicher und
   Bestimmen der Werte von c/d, a/c und a/d, der Mittelwerte von $\bar{c}$ und $\bar{d}$ der rückwärtigen Randlängen c bzw. der vorderen Randlänge d und der Standard-Abweichungen $\sigma_c$ und $\sigma_d$ der Werte c bzw. d;
   (ii) Messen der Werte a, c und d aus einer Vielzahl von Profil-Röntgenaufnahmen, welche von Wirbelkörpern mit mindestens einer Deformation erhalten wurden, und Speichern dieser Messungen in dem Speicher und Bestimmung der Werte c/d, a/c, a/d und $\bar{c}$, $\bar{d}$, $\sigma_c$ und $\sigma_d$;
   (iii) Erstellen eines Beurteilungsstandards aus den in den Schritten (i) und (ii) erhaltenen Daten zur Bestimmung der Art der Deformation entsprechend einer Klassifizierung "deformationsloser" Wirbel, "keilförmiger" Wirbel, "invers-keilförmiger" Wirbel, "Fisch"-Wirbel und "flacher" Wirbel;
   (iv) Messen von a, c und d aus einer Profil-Röntgenaufnahme des zu beurteilenden Wirbelkörpers, Speichern der Meßwerte in dem Speicher und Bestimmen von c/d, a/c und a/d; und
   (v) Klassifizieren der Deformation des zu beurteilenden Wirbelkörpers nach dem c/d-Wert im Falle von "keilförmigen" und "invers-keilförmigen" Wirbeln,
   nach den Werten $\bar{c}$, $\bar{d}$, $\sigma_c$, $\sigma_d$ und c/d im Fall eines "flachen" Wirbels und
   nach den Werten $\bar{c}$, $\bar{d}$, $\sigma_c$, $\sigma_d$, a/c, a/d und c/d im Falle von "deformationslosen" und "Fisch"-Wirbel,
   um die Deformation zu beurteilen und die Auswirkung einer Therapie auf diesen zu beurteilenden Wirbelkörper zu beobachten.

**2.** Verfahren nach Anspruch 1, worin der Schritt (v) gemäß den folgenden Teilschritten ausgeführt wird:

(i) $c/d \geq \beta_1$ ... keilförmiger Wirbel (oder frühere keilförmige Fraktur)

(ii) $c/d \leq \beta_2$ ... invers-keilförmiger Wirbel (oder frühere invers-keilförmige Fraktur)

(iii) $c < \bar{c} - \beta_3 \, \sigma_c$, $d < \bar{d} - \beta_4 \, \sigma_d$ und $\beta_2 < c/d < \beta_1$ ... flacher Wirbel (oder Kompressionsfraktur)

(iv) $\beta_2 < c/d < \beta_1$, $a/c \leq \beta_5$, $a/d \leq \beta_6$ und $c \geq c - \beta_3 \, \sigma_c$ und/oder $d \geq - \beta_4 \, \sigma_d$ ... Fisch-Wirbel (oder zentrale bikonkave Fraktur)

(v) $\beta_2 < c/d < \beta_1$, $c \geq \bar{c} - \beta_3 \, \sigma_c$ und/oder $d \geq d - \beta_4 \, \sigma_d$ und $a/c > \beta_5$ und/oder $a/d > \beta_6$ ... deformationslos (oder keine Fraktur),

wobei $\beta_1$ bis $\beta_6$ ausgewählt sind wie folgt:

$1{,}25 \leq \beta_1 \leq 1{,}55$, $0{,}6 \leq \beta_2 \leq 0{,}8$, $1 \leq \beta_3 \leq 2{,}5$, $1 \leq \beta_4 \leq 2{,}5$, $0{,}65 \leq \beta_5 \leq 0{,}9$ und $0{,}65 \leq \beta_6 \leq 0{,}9$.

**3.** Verfahren nach Anspruch 2, worin $\beta_1$ bis $\beta_6$ definiert sind als:

$1{,}33 \leq \beta_1 \leq 1{,}5$, $0{,}65 \leq \beta_2 \leq 0{,}75$, $1{,}25 \leq \beta_3 \leq 2{,}25$, $1{,}25 \leq \beta_4 \leq 2{,}25$, $0{,}7 \leq \beta_5 \leq 0{,}85$ und $0{,}7 \leq \beta_6 \leq 0{,}85$.

**4.** Verfahren nach Anspruch 3, worin $\beta_1$ 1,4, $\beta_2$ 0,7, $\beta_3$ 2, $\beta_4$ 1,5, $\beta_5$ 0,8 und $\beta_6$ 0,8 ist.

**5.** Verfahren nach einem der Ansprüche 2 bis 4, worin zumindest einer der Werte $\beta_1$ bis $\beta_6$ durch eine diskriminierende Funktion bestimmt wird, welche zwei Gruppen mit einer oder zwei Variablen unterscheiden kann, unter Verwendung von a, b, c und d oder einem Verhältnis hiervon, welche durch Messungen an mindestens 5 Profil-Röntgenaufnahmen von der zwei Typen, ausgewählt aus den vier Typen "keilförmiger" Wirbel "Fisch"-Wirbel, "flacher" Wirbel und "deformationsloser" Wirbel, erhalten wurden.

**6.** Verfahren nach einem der voranstehenden Ansprüche, worin die Messungen mittels eines Maßstabes, eines Digitalisierers oder einer automatischen Vorrichtung vorgenommen werden, welche die Längen bei einer Bildaufarbeitung unter Speichern der Röntgenaufnahme mittels automatischer Röntgenaufnahmeeinlesung müßt.

**7.** Gerät zum Beurteilen eines Wirbelkörpers auf Deformationen gemäß dem Verfahren nach Anspruch 1, wobei das Gerät umfaßt:

(i) eine Eingabeeinheit zum Eingeben von Werten für zumindest die mittige Länge a, die rückwärtige Randlänge c und die vordere Randlänge d aus einer Profil-Röntgenaufnahme eines zu beurteilenden Wirbelkörpers;

(ii) eine Arithmetik-Einheit zum Durchführen der Berechnung von c/d, a/c, a/d, der Mittelwerte c, $\bar{d}$ von c und d und der Standardabweichungen $\sigma_c$ und $\sigma_d$ der Werte c und d, welche notwendig sind, um die Art der Deformation entsprechend der Klassifikation, die besteht aus "keilförmiger Wirbel", "invers-keilförmiger Wirbel", "Fisch-Wirbel", "flacher Wirbel" und "deformationsloser Wirbel" unter Verwendung der Eingabewerte zu bestimmen;

(iii) eine Diskriminator-Einheit zum Unterscheiden von "keilförmigen" und "invers-keilförmigen" Wirbeln aus c/d, "flachen" Wirbeln aus $\bar{c}$, $\bar{d}$, $\sigma_c$, $\sigma_d$ und c/d und

"deformationslose" und "Fisch"-Wirbel aus c, d, $\sigma_c$, $\sigma_d$, a/c, a/d und c/d;

(iv) eine Eingabeeinheit für den Beurteilungsstandard, der für die Berechnung und Unterscheidung notwendig ist; und

(v) eine Ausgabeeinheit zum Ausgeben des Beurteilungsergebnisses;

**8.** Verwendung des Geräts nach Anspruch 7 zum Ausführen des Verfahrens nach einem der Ansprüche 2 bis 6.

**Revendications**

**1.** Procédé pour évaluer la déformation d'un corps vertébral dans un sujet, comprenant les étapes consistant :

(i) à mesurer d'une longueur centrale a, une longueur du bord arrière c et une longueur du bord avant d à partir de plusieurs radiographies de profil obtenues à partir de corps vertébraux ayant substantiellement aucune déformation à une partie correspondant à une partie dudit corps vertébral à évaluer, à stocker les mesures dans une mémoire, et à déterminer les valeurs de c/d, a/c et a/d, les valeurs moyennes c et d respectivement des longueurs de bord arrière c et des longueurs de bord avant d, et les déviations de référence $\sigma_c$ et $\sigma_d$ respectivement des valeurs c et d;

(ii) à mesurer a, c et d à partir de plusieurs radiographies de profil obtenues à partir de corps vertébraux ayant au moins une déformation, à stocker les mesures dans ladite mémoire, et à déterminer les valeurs de c/d, a/c, a/d et $\bar{c}$, $\bar{d}$, $\sigma_c$ et $\sigma_d$;

(iii) à préparer une référence d'évaluation à partir des données obtenues dans les étapes (i) et (ii) afin de déterminer la nature de la déformation selon une classification consistant en vertèbres "sans déformation", vertèbres "cunéiformes", vertèbres "cunéiformes inversées", vertèbres "en poisson", et vertèbres "plates";

(iv) à mesurer a, c et d à partir d'une radiographie de profil dudit corps vertébral à évaluer, à stocker les mesures dans ladite mémoire, et à déterminer c/d, a/c et a/d; et

(v) à classifier la déformation du corps vertébral à évaluer à partir de c/d dans le cas de vertèbres "cunéiformes" et de vertèbres "cunéiformes inversées", à partir de $\bar{c}$, $\bar{d}$, $\sigma_c$ et $\sigma_d$, et c/d dans le cas de vertèbres "plates", et à partir de $\bar{c}$, $\bar{d}$, $\sigma_c$, $\sigma_d$, a/c, a/d et c/d dans le cas de vertèbres "sans déformation" et de vertèbres "en poisson", afin d'évaluer la déformation et de contrôler l'effet thérapeutique sur ledit corps vertébral à évaluer.

2. Procédé selon la revendication 1, dans lequel on effectue ladite étape (v) selon les sous-étapes suivantes :

(i) c/d ≧ $\beta_1$ ... vertèbres cunéiformes (ou fracture en forme de coin inversé antérieure)

(ii) c/d ≦ $\beta_2$ ... vertèbres cunéiformes inversées (ou fracture en forme de coin inversé antérieure)

(iii) c < $\bar{c}$ - $\beta_3$ $\sigma_c$, d < d - $\beta_4$ $\sigma_d$, et $\beta_2$ < c/d < $\beta_1$ ... vertèbres plates (ou fracture en compression)

(iv) $\beta_2$ < c/d < $\beta_1$, a/c ≦ $\beta_5$, a/d ≦ $\beta_6$ et c ≧ $\bar{c}$ -$\beta_3$ $\sigma_c$ et/ou d ≧ $\bar{d}$ - $\beta_4$ $\sigma_d$ ... vertèbres en poisson (ou fracture biconcave centrale)

(v) $\beta_2$ < c/d < $\beta_1$, c ≧ $\bar{c}$ - $\beta_3$ $\sigma_c$, et/ou d ≧ $\bar{d}$ - $\beta_4$ $\sigma_d$, et a/c > $\beta_5$ et/ou a/d > $\beta_6$ ... Sans déformation (ou sans fracture), où $\beta_1$ à $\beta_6$ sont choisis comme suit : 1,25 ≦ $\beta_1$ ≦ 1,55, 0,6 ≦ $\beta_2$ ≦ 0,8, 1 ≦ $\beta_3$ ≦ 2,5, 1 ≦ $\beta_4$ ≦ 2,5, 0,65 ≦ $\beta_5$ ≦ 0,9, et 0,65 ≦ $\beta_6$ ≦ 0,9.

3. Procédé selon la revendication 2, dans lequel $\beta_1$ à $\beta_6$ sont tels que 1,33 ≦ $\beta_1$ ≦ 1,5, 0,65 ≦ $\beta_2$ ≦ 0,75, 1,25 ≦ $\beta_3$ ≦ 2,25, 1,25 ≦ $\beta_4$ ≦ 2,25, 0,7 ≦ $\beta_5$ ≦ 0,85, et 0,75 ≦ $\beta_6$ ≦ 0,85.

4. Procédé selon la revendication 3, dans lequel $\beta_1$ est 1,4, $\beta_2$ 0,7, $\beta_3$ 2, $\beta_4$ 1,5, $\beta_5$ 0,8, et $\beta_6$ 0,8.

5. Procédé selon l'une quelconque des revendications 2 à 4, dans lequel au moins un des $\beta_1$ à $\beta_6$ sont déterminés par une fonction discriminante capable de disoriminer deux groupes avec une ou deux variables en utilisant deux de a, b, c et d ou de leur rapport, qui sont obtenus en mesurant au moins 5 radiographies de profil dans chacun des deux types choisis parmi quatre types, "vertèbres cunéiformes", vertèbres en poisson", "vertèbres plates" et "sans déformation".

6. Procédé selon l'une quelconque des revendications précédentes, dans lequel on effectue la mesure par un moyen de graduation, un moyen de conversion numérique, ou un moyen pour la mesure automatique des longueurs à partir du traitement d'images par mémorisation de la radiographie à l'aide d'un moyen d'entrée automatique de radiographies.

7. Appareil pour évaluer la déformation d'un corps vertébral selon le procédé de la revendication 1, l'appareil comprenant (i) un moyen d'entrée pour introduire des valeurs d'du moins la longueur centrale a, la longueur du bord arrière c et la longueur du bord avant d à partir d'une radiographie de profil d'un corps vertébral à évaluer, (ii) un moyen arithmétique pour exécuter le calcul de c/d, a/c, a/d, les valeurs moyennes $\bar{c}$, $\bar{d}$ de c et d et les déviations de référence $\sigma_c$ et $\sigma_d$ des valeurs c et d, respectivement, nécessaires pour déterminer la nature de la déformation selon une classification consistant en vertèbres "cunéiformes" vertèbres "cunéiformes inversées", vertèbres "en poisson", vertèbres "pistes" et "sans déformation" en utilisant lesdites valeurs d'entrée, (iii) un moyen pour discriminer les vertèbres "cunéiformes" et des vertèbres "cunéiformes inversées" à partir de c/d, des vertèbres "plates" à partir de $\bar{c}$, $\bar{d}$, $\sigma_c$, $\sigma_d$ et c/d, et des vertèbres "sans déformation" et "en poisson" à partir de $\bar{c}$, $\bar{d}$, $\sigma_c$, $\sigma_d$, a/c, a/d et c/d, (iv) un moyen pour entrer la référence d'évaluation nécessaire audit calcul et à ladite discrimination, et (v) un moyen de sortie pour sortir le résultat de l'évaluation.

8. Utilisation de l'appareil selon la revendication 7, pour la mise en oeuvre du procédé selon l'une quelconque des revendications 2 à 6.

*Fig. 1*

*Fig. 2*

# *Fig. 3*

NO DEFOMATION

WEDGE-SHAPED
VERTEBRAL

FISH VERTEBRAL       FLAT VERTEBRAL

INVERSE
WEDGE-SHAPED
VERTEBRAL

# *Fig. 4*

# Fig. 5

```
        ┌─────────────────────────────┐
        │  DISCRIMINATING  STANDARD   │
        │      INPUT   MEANS          │
        └─────────────────────────────┘
              │                 │
              ▼                 ▼
┌──────────┐  ┌─────────────┐  ┌─────────────────┐  ┌──────────────┐
│  INPUT   │→ │ ARITHMETIC  │→ │ DISCRIMINATING  │→ │ OUTPUT MEANS │
│  MEANS   │  │   MEANS     │  │     MEANS       │  │              │
└──────────┘  └─────────────┘  └─────────────────┘  └──────────────┘
```

EP 0 245 098 B1

## Fig. 6

# Fig. 7

ARITHMETIC MEANS FOR DISCRIMINATING STANDARD IN CASE OF TWO GROUPS WITH ONE VARIABLE

X-RAY IMAGE OF EACH VERTEBRAL BODY

INPUT MEANS

ARITHMETIC MEANS OF CONSTANTS AND VARIABLES IN DISCRIMINANT FUNCTION IN CASE OF 2-4 GROUPS WITH 2-4 VARIABLES

MEMORY MEANS

DISCRIMINATING STANDARD INPUT MEANS

DISCRIMINANT FUNCTION INPUT MEANS

DISCRIMINATING STANDARD INPUT MEANS

X-RAY IMAGE TO BE JUDGED

INPUT MEANS

ARITHMETIC MEANS FOR DISCRIMINATING VALUE

DISCRIMINATING MEANS

OUTPUT MEANS

EP 0 245 098 B1

# Fig. 8

CRT ( DISPLAY )

DIGITIZER   IMAGE PC   PERSONAL   MEMORY   PRINTER
(IMAGE TREATMENT) COMPUTOR

EP 0 245 098 B1

# Fig. 9

SUPPORT  MOVABLE ARM  CRT

TV CAMERA

LIGHT BOX

AMPLIFIYER

IMAGE PC  PERSONAL COMPUTOR  MEMORY  PRINTER